# FASCICULE DE BREVET EUROPEEN

(11) **EP 1 585 448 B1**
(45) Date de publication et mention de la délivrance du brevet: **30.11.2011**
(21) Numéro de dépôt: 03815954.7
(22) Date de dépôt: 29.12.2003
(51) Int. Cl.: A61B 17/70, A61L 27/38, A61F 2/46

(54) **ENSEMBLE DE TRAITEMENT DE LA DEGENERESCENCE D'UN DISQUE INTERVERTEBRAL**
SYSTEM ZUR BEHANDLUNG DER DEGENERATION EINER BANDSCHEIBE
SYSTEM FOR TREATMENT OF DEGENERATION OF AN INTERVERTEBRAL DISC

(30) Priorité: 20.01.2003 FR 0300555
(43) Date de publication de la demande: 19.10.2005
(73) Titulaire: ZIMMER SPINE, 33000 Bordeaux (FR)
(72) Inventeur: MEUNIER, Alain, F-75020 PARIS (FR); SENEGAS, Jacques, F-33700 MERIGNAC (FR); LE COUEDIC, Régis, F-78570 ANDRESY (FR)
(74) Mandataire: Intès, Didier Gérard André
(86) Numéro de dépôt international: PCT/FR2003/003929
(87) Numéro de publication internationale: WO 2004/073532

(56) Documents cités:
- FR-A- 2 775 183
- US-A1- 2002 151 981
- US-B1- 6 344 058
- US-B1- 6 419 702

## Description

"Ensemble de traitement de la dégénérescence d'un disque intervertébral"

La présente invention a pour objet un ensemble de traitement de la dégénérescence d'un disque intervertébral.

Comme cela est bien connu, le disque intervertébral est un moyen d'union des corps vertébraux entre eux dont la forme est celle d'une lentille biconvexe qui s'adapte et s'attache aux surfaces articulaires des corps des vertèbres. Ils sont constitués de deux parties : une partie périphérique très dure de texture très serrée et une partie centrale qui est une substance molle et gélatineuse.

Pour différentes raisons, des pathologies du disque intervertébral peuvent entraîner la dégénérescence plus ou moins importante de ce disque qui alors ne peut plus remplir sa fonction normale entre les vertèbres.

En face de cette lésion ou dégénérescence du disque intervertébral, il est nécessaire d'appliquer une technique chirurgicale pour tenter d'obtenir la régénération de ce disque et donc son comportement biomécanique normal.

Une technique pour tenter d'obtenir cette régénération consiste à procéder à l'implantation dans le disque lésé de cellules destinées à sa régénération, ces cellules pouvant être de la même nature que celle constituant le disque ou d'une autre nature.

Toutefois, on peut douter de l'efficacité de cette technique qui ne ferait qu'introduire de nouvelles cellules dans un environnement biomécanique déjà dégradé, les soumettant à de trop fortes contraintes, compromettant ainsi leur survie à long terme. Il apparaît donc qu'un tel traitement a peu de chance de produire le résultat escompté, c'est-à-dire la régénération du disque intervertébral.

Il est donc important de définir des moyens permettant d'obtenir effectivement la régénération du disque intervertébral de façon pérenne. C'est l'objet de la présente invention.

Les inventeurs ont mis en évidence le fait qu'après l'implantation dans le disque intervertébral lésé des cellules de régénération, la lésion ou la nécrose des cellules constituant le disque ne disparaissait pas ou ne disparaissait pas de façon suffisante en raison de la pression mécanique exercée sur ce disque par les vertèbres qui l'entourent. D'ailleurs, les inventeurs ont également mis en évidence qu'une partie de la dégénérescence des cellules du disque intervertébral pourrait avoir une origine purement mécanique ou que cette origine purement mécanique pouvait avoir créé des conditions favorables à la dégénérescence de ce disque pour d'autres lésions biologiques.

Comme on l'a indiqué précédemment, l'objet de l'invention est atteint par un ensemble de traitement de la dégénérescence d'un disque intervertébral lésé disposé entre deux vertèbres qui se caractérise en ce qu'il comprend des cellules analogues ou non à celles du disque intervertébral et implantables dans ledit disque ; et un implant intervertébral comportant une cale intervertébrale destinée à être disposée entre lesdites vertèbres pour limiter les contraintes appliquées auxdits disques et des moyens de fixation de ladite cale sur lesdites vertèbres.

On comprend que d'une part les cellules implantées dans le disque intervertébral vont permettre la régénération des cellules du disque intervertébral et que cette régénération sera possible grâce à la présence et à la mise en place de l'implant intervertébral dont la cale est disposée entre les apophyses des vertèbres et maintient donc un écartement entre celles-ci et limitent ainsi les contraintes mécaniques appliquées au disque intervertébral en cours de traitement.

Les cellules implantables utilisées peuvent être obtenues par différents procédés tels que le prélèvement sur un disque intervertébral du malade, le prélèvement dans la moelle osseuse du malade de cellules souches adultes ou encore par prélèvement de cellules souches embryonnaires.

En outre, l'ensemble de traitement comporte avantageusement des moyens d'injection des cellules dans le disque, ces moyens d'injection pouvant être du type seringue à canule.

De préférence également, la cale de l'implant intervertébral comprend une partie centrale et deux parties d'extrémité, chaque partie d'extrémité comportant une gorge limitée par deux ailes, ladite gorge étant apte à recevoir l'apophyse épineuse d'une des vertèbres entourant le disque à traiter.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées sur lesquelles :
- la figure 1 montre l'ensemble de traitement de la dégénérescence d'un disque intervertébral conforme à l'invention ;
- la figure 2 est une vue en perspective d'un implant intervertébral utilisable dans la présente invention ; et
- la figure 3 est une vue en coupe verticale de l'implant montré sur la figure 2.

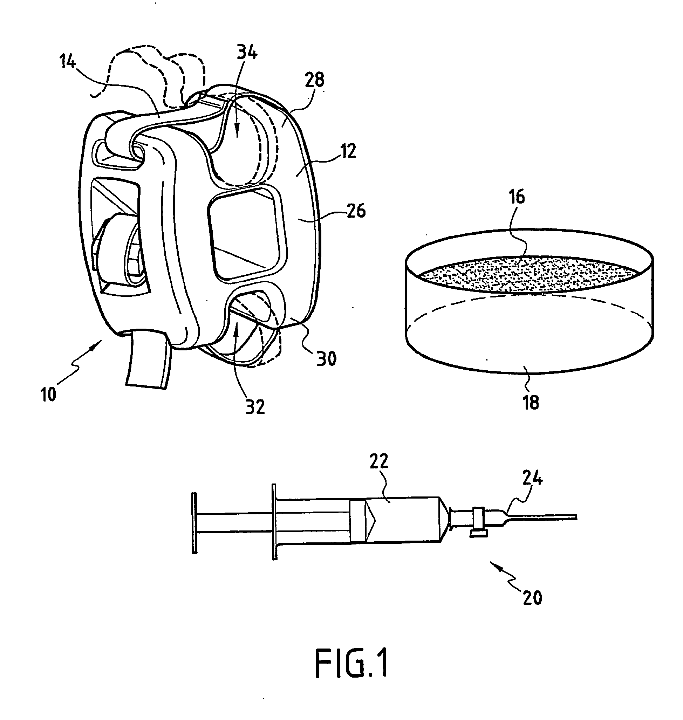

Sur la figure 1, on a représenté de façon simplifiée, d'une part un implant intervertébral 10 comportant une cale 12 et des moyens de fixation 14 ; des cellules injectables pour la régénération du disque intervertébral, ces cellules étant symboliquement référencées 16 et montrées dans une boîte de culture 18 et enfin les moyens d'injection des cellules 16 dans le disque intervertébral 20. Les moyens d'injection 20 sont constitués dans ce mode particulier de l'invention d'un corps de seringue 22 et d'une canule 24.

Ainsi qu'on l'a déjà expliqué selon l'invention, l'implant intervertébral 10 constituant une partie de l'ensemble de traitement est destiné à permettre la fixation d'un écartement donné entre les vertèbres entourant le disque intervertébral à traiter et à limiter ainsi les contraintes mécaniques appliquées à ce disque. Par ailleurs, cet ensemble de traitement comporte les cellules injectables pour la thérapie de la cellule, l'injection ou l'implantation de ces cellules pouvant avoir lieu en même temps que la mise en place de l'implant ou après, ainsi que l'on l'expliquera ultérieurement.

Les implants intervertébraux sont en eux mêmes bien connus pour leur fonction d'écartement et de solidarisation des vertèbres par leurs apophyses. De tels implants intervertébraux sont notamment décrits dans FR-A- 2 775 183 et dans les demandes de brevets PCT du demandeur et en particulier dans les demandes de brevets WO 02/051326 et PCT/FR02/00888.

D'une manière générale, ainsi qu'on le décrira ultérieurement et ainsi que le montre la figure 1, ces implants sont constitués par une cale 12 qui comprend une partie centrale 26 et deux parties d'extrémité 28 et 30. Les parties d'extrémité 28 et 30 définissent des gorges 32 et 34 destinées à recevoir les apophyses épineuses des vertèbres dont on veut maintenir sensiblement constante la distance mutuelle. D'une manière générale, de préférence, la partie centrale de la cale 12 présente des possibilités de déformation élastique afin de permettre un déplacement relatif des vertèbres tout en assurant leur écartement convenable.

En ce qui concerne les cellules implantables utilisables pour la régénération du disque intervertébral, elles peuvent avoir différentes origines :
il peut s'agir de cellules prélevées sur le malade au niveau du disque lui-même et mises en culture dans un milieu adéquat (éventuellement en tridimensionnel) telle que (bille d'alginate, structure poreuse en PLA/TGA, mousse de colagène) ;

On peut également utiliser des cellules souches adultes autologues prélevées au niveau de la moelle osseuse par exemple dans la crête iliaque, puis placées dans un milieu de culture identique à ceux qui ont été mentionnés ci-dessus.

Enfin, on peut utiliser des cellules souches embryonnaires traitées de la même manière que cela a été indiqué précédemment.

Ces cellules peuvent être implantées dans le disque intervertébral à traiter en même temps que la pose de l'implant intervertébral ou bien faire l'objet d'une opération à part entière ayant lieu avant ou après la pose de l'implant intervertébral. Dans tous les cas, on obtient des résultats similaires.

Dans le cas d'une intervention chirurgicale au cours de laquelle l'implantation des cellules dans le disque intervertébral et la mise en place de l'implant intervertébral sont simultanées, les étapes opératoires sont les suivantes :
- en ce qui concerne l'installation du patient devant subir l'opération, il est placé en décubitus ventral. Une position de lordose lombaire physiologique est souhaitable.
- le chirurgien procède à l'exposition des apophyses épineuses et à la désinsertion du ligament surépineux.
- il prépare notamment par curetage, les apophyses épineuses des vertèbres concernées ;
- il procède ensuite à l'injection dans le disque intervertébral des cellules de culture.

Les préparatifs de l'étape d'injection des cellules dans le disque intervertébral dépendent du type du milieu de culture qui a été utilisé. En effet, il peut être nécessaire de les séparer de ces milieux préalablement à leurs implantations dans le disque.

Ensuite, le chirurgien mesure l'espace entre les apophyses épineuses des vertèbres afin de déterminer la taille de la cale de l'implant intervertébral à mettre en place. Enfin, il procède à la mise en place de l'implant selon la technique opératoire correspondant à l'implant choisi puis il referme la voie d'abord.

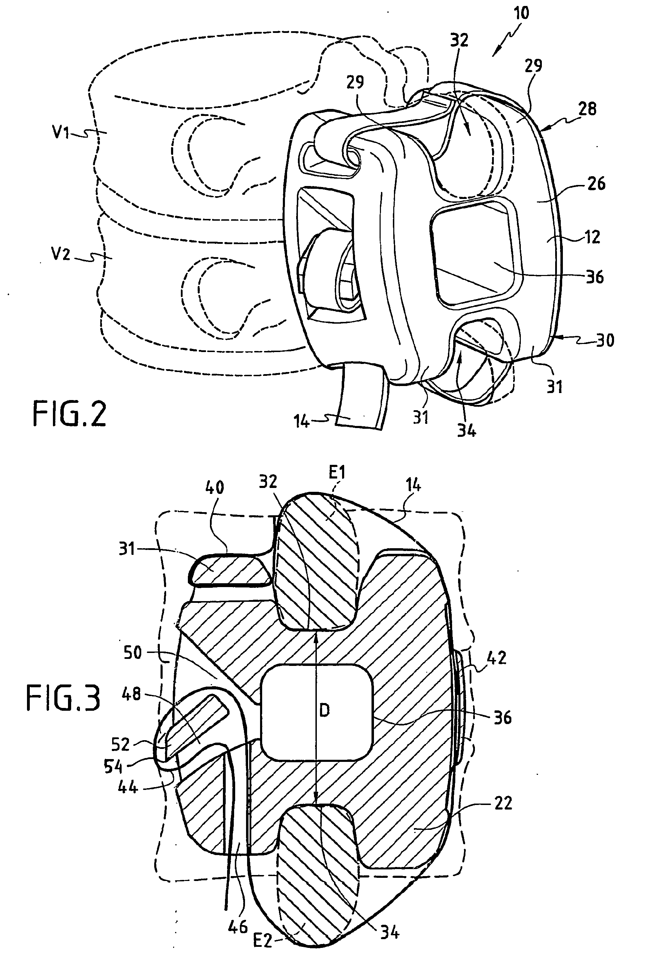

Sur les figures 2 et 3, on a représenté un implant intervertébral particulièrement bien adapté à la mise en oeuvre de l'invention. Cependant, il va de soi que d'autres types d'implants intervertébraux pourraient être utilisés. L'implant décrit correspond à la demande de brevet PCT WO 02/051326.

L'implant 10 est constitué par une cale 12 et des moyens de fixation 14. La cale 12 comprend une partie centrale 26 et deux parties d'extrémité 28 et 30. Les parties d'extrémité 28 et 30 comportent chacune une paire d'ailes 29 et 31 définissant deux gorges parallèles 32 et 34. Chaque gorge 32, 34 est destinée à recevoir l'apophyse épineuse E₁, E₂ des vertèbres V₁ et V₂ entre lesquelles la cale 12 est insérée.

De préférence, la cale 12, qui est réalisée avec un matériau rigide, comporte dans sa partie centrale 26 un évidement 36, par exemple, de section droite rectangulaire, qui s'étend parallèlement aux gorges 32 et 34. Cet évidement 36 donne une certaine élasticité à la partie centrale 26 de la cale, ce qui autorise un certain déplacement relatif des vertèbres V₁ et V₂. Cependant, en moyenne, la distance entre les deux vertèbres est imposée par la distance D entre le fond des gorges 32 et 34.

Pour maintenir les apophyses E₁ et E₂ dans les gorges 32 et 34 de la cale 12, l'implant comprend des moyens de fixation constitués dans l'exemple particulier décrit, par une bande 14.

La bande 14 présente une première extrémité 40 qui est fixée sur une aile 29 de la gorge 32. La partie couvrante 42 de la bande 14 passe en regard des gorges 32 et 34 et sa deuxième extrémité 44 passe dans des fentes 46, 48 et 50 ménagées dans l'aile 31 et dans la partie médiane 26 de la cale. La portion 52 de cale définie par les fentes 48 et 50 présente une extrémité 54 effilée permettant un auto-blocage de la bande 14.

## Revendications

1. Ensemble de traitement de la dégénérescence d'un disque intervertébral lésé disposé entre deux vertèbres, **caractérisé en ce qu'**il comprend :
- des cellules (16) analogues ou non à celles du disque intervertébral et implantables dans ledit disque ; et
- un implant intervertébral (10) comportant :
une cale intervertébrale (12) destinée à être disposée entre lesdites vertèbres pour limiter les contraintes appliquées audit disque ; et
des moyens de fixation (14) de ladite cale sur lesdites vertèbres.

2. Ensemble de traitement selon la revendication 1, **caractérisé en ce que** lesdites cellules (16) sont telles qu'obtenues par prélèvement sur un disque intervertébral du malade et par culture desdites cellules prélevées.

3. Ensemble de traitement selon la revendication 1, **caractérisé en ce que** lesdites cellules (16) sont telles qu'obtenues par prélèvement dans la moelle osseuse du malade de cellules souches adultes analogues et par culture desdites cellules.

4. Ensemble de traitement selon la revendication 1, **caractérisé en ce que** lesdites cellules (16) sont telles qu'obtenues par prélèvement de cellules souches embryonnaires et par culture desdites cellules.

5. Ensemble de traitement selon l'une quelconque des revendications 1 à 4, **caractérisé en ce qu'**il comprend en outre des moyens d'injection (20) desdites cellules implantables (16) dans le disque intervertébral du malade.

6. Ensemble de traitement selon l'une quelconque des revendications 1 à 5, **caractérisé en ce que** ladite cale (12) dudit implant intervertébral comprend une partie centrale (26) et deux parties d'extrémité (28, 30), chaque partie d'extrémité comportant une gorge (32, 34) limitée par deux ailes (29, 31), ladite gorge étant apte à recevoir l'apophyse épineuse (E₁, E₂) d'une vertèbre (V₁, V₂).

7. Ensemble de traitement selon la revendication 6, **caractérisé en ce que** ladite partie centrale (26) de la cale (12) est élastiquement déformable.

8. Ensemble de traitement selon l'une quelconque des revendications 6 et 7, **caractérisé en ce que** lesdits moyens de fixation (14) de l'implant intervertébral comprennent au moins une bande (14) solidarisable sur ladite cale (12) et entourant l'apophyse (E₁, E₂) desdites vertèbres pour maintenir lesdites apophyses dans lesdites gorges (32, 34).

## Claims

1. A kit for treating degeneration of a damaged intervertebral disk disposed between two vertebrae, the kit being **characterized in that** it comprises:
- cells (16) optionally analogous to those of the intervertebral disk, and suitable for being implanted in said disk; and
- an intervertebral implant (10) comprising:
- an intervertebral spacer (12) intended to be placed between said vertebrae in order to limit the stresses applied to said disk; and
- fastener means (14) for fastening said spacer to said vertebrae.

2. A treatment kit according to claim 1, **characterized in that** said cells (16) are as obtained by being taken from an intervertebral disk of the patient and culturing said cells as taken.

3. A treatment kit according to claim 1, **characterized in that** said cells (16) are such as obtained by taking autologous adult stem cells from the bone marrow of the patient and by culturing said cells.

4. A treatment kit according to claim 1, **characterized in that** said cells (16) are such as obtained by taking embryonic stem cells and by culturing said cells.

5. A treatment kit according to any one of claims 1 to 4, **characterized in that** it further comprises injector means (20) for injecting said implantable cells (16) into the intervertebral disk of the patient.

6. A treatment kit according to any one of claims 1 to 5, **characterized in that** said spacer (12) of said intervertebral implant comprises a central portion (26) and two end portions (28, 30), each end portion having a groove (32, 34) defined by two limbs (29, 31), said groove being adapted for receiving the spinous process (E₁, E₂) of a vertebra (V₁, V₂).

7. A treatment kit according to claim 6, **characterized in that** said central portion (26) of the spacer (12) is elastically deformable.

8. A treatment kit according to any one of claims 6 and 7, **characterized in that** said fastener means (14) for fastening the intervertebral implant comprise at least one strip (14) securable to said spacer (12) and surrounding the process (E₁, E₂) of said vertebrae so as to hold said processes in said grooves (32, 34).

## Patentansprüche

1. Anordnung zur Behandlung der Degeneration einer zwischen zwei Wirbeln angeordneten geschädigten Bandscheibe, **dadurch gekennzeichnet, daß** sie umfaßt:
- Zellen (16), die zu denjenigen der Bandscheibe analog sind oder nicht und die in die Scheibe implantierbar sind, sowie
- ein Zwischenwirbelimplantat (10) umfassend:
einen Zwischenwirbelkeil (12), der dazu bestimmt ist, zwischen den Wirbeln angeordnet zu werden, um die Belastungen auf die Scheibe zu begrenzen, und
Mittel zum Befestigen (14) des Keils an den Wirbeln.

2. Behandlungsanordnung nach Anspruch 1, **dadurch gekennzeichnet, daß** die Zellen (16) derart sind, wie sie durch Entnahme an einer Bandscheibe des Patienten und durch Kultivieren der entnommenen Zellen erhalten werden.

3. Behandlungsanordnung nach Anspruch 1, **dadurch gekennzeichnet, daß** die Zellen (16) derart sind, wie sie durch Entnahme von analogen adulten Stammzellen aus dem Knochenmark des Patienten und durch Kultivieren der Zellen erhalten werden.

4. Behandlungsanordnung nach Anspruch 1, **dadurch gekennzeichnet, daß** die Zellen (16) derart sind, wie sie durch Entnahme von embryonalen Stammzellen und durch Kultivieren der Zellen erhalten werden.

5. Behandlungsanordnung nach einem der Ansprüche 1 bis 4, **dadurch gekennzeichnet, daß** sie ferner Mittel zum Injizieren (20) der implantierbaren Zellen (16) in die Bandscheibe des Patienten umfaßt.

6. Behandlungsanordnung nach einem der Ansprüche 1 bis 5, **dadurch gekennzeichnet, daß** der Keil (12) des Zwischenwirbelimplantats einen mittleren Teil (26) und zwei endseitige Teile (28, 30) umfaßt, wobei jeder endseitige Teil eine durch zwei Flügel (29, 31) begrenzte Nut (32, 34) umfaßt, wobei die Nut geeignet ist, den Dornfortsatz (E₁, E₂) eines Wirbels (V₁, V₂) aufzunehmen.

7. Behandlungsanordnung nach Anspruch 6, **dadurch gekennzeichnet, daß** der mittlere Teil (26) des Keils (12) elastisch verformbar ist.

8. Behandlungsanordnung nach einem der Ansprüche 6 und 7, **dadurch gekennzeichnet, daß** die Befestigungsmittel (14) des Zwischenwirbelimplantats wenigstens ein Band (14) umfassen, das mit dem Keil (12) fest verbindbar ist und das den Fortsatz (E₁, E₂) der Wirbel umgreift, um die Fortsätze in den Nuten (32, 34) zu halten.
